# EUROPEAN PATENT APPLICATION

(11) **EP 2 450 450 A1**
(43) Date of publication of application: **09.05.2012**
(21) Application number: 10190539.6
(22) Date of filing: 09.11.2010
(51) Int. Cl.: C12P 7/06, C12M 1/04, C07C 11/04

(54) **Process and apparatus for producing ethylene via preparation of syngas**

(71) Applicant: Ineos Commercial Services UK Limited, Hampshire SO43 7FG (GB)
(72) Inventor: The designation of the inventor has not yet been filed
(74) Representative: Rickard, Timothy Mark Adrian

(57) **Abstract**

A process for producing ethylene from a carbonaceous containing feedstock the process comprising the steps of
a) reforming the carbonaceous containing feedstock into a first product stream comprising carbon dioxide, carbon monoxide and hydrogen;
b) fermenting the first product stream with an acetogenic anaerobic bacteria to produce a second product stream comprising ethanol;
c) concentrating ethanol from the second product stream into a third product stream; and optionally
d) dehydrating ethanol in the third product stream to produce ethylene.

## Description

This invention relates to processes and apparatus for producing olefin derivatives. More especially the invention relates to the production of olefin derivatives such as ethylene, polyethylene, ethylbenzene, ethylene oxide, ethylene glycol, ethanolamine, crown ethers and vinyl chloride monomer via bacterial fermentation.

Many olefin derivatives are produced from oil. It is desirable to be able to produce them from a wider range of starting materials.

WO2010/012 946 (Arkema) describes a process for making polyethylene from biomass. The biomass is fermented to convert cellulose, sugars and starches into ethanol using the yeast *Saccharomyces cerivisiae.* The ethanol is dehydrated to produce a dilute solution of ethanol in water which is concentrated. The ethanol is then dehydrated to produce ethylene which is polymerised. As explained in WO2010/012 946 the process is suitable for small scale plants not large scale industrial processes.

An aspect of the invention seeks to overcome the problems with the prior art and provide a large-scale process for producing ethylene and olefin derivatives via a fermentation step.

Processes are known for producing ethylene and olefin derivatives from methane as will be described hereinafter but the yield of ethylene is low. The invention seeks to produce ethylene in higher yield.

According to the invention there is provided a process for producing ethylene from a carbonaceous containing feedstock. The process comprising the steps of
a) reforming the carbonaceous containing feedstock into a first product stream comprising carbon dioxide, carbon monoxide and hydrogen;
b) fermenting the first product stream with an acetogenic anaerobic bacteria to produce a second product stream comprising ethanol;
c) concentrating ethanol from the second product stream into a third product stream; and optionally
d) dehydrating the ethanol in the third product stream to produce ethylene.

According to the invention there is further provided apparatus for producing ethylene comprising
i) a reformer for reforming a carbonaceous feedstock into a first product stream comprising carbon dioxide, carbon monoxide and hydrogen;
ii) a fermenter for fermenting the first product stream into a second product stream comprising ethanol;
iii) means for concentrating ethanol from the second product stream; and optionally
iv) a dehydrator for dehydrating the ethanol to ethylene.

In outline a carbonaceous feedstock such as natural gas, shale gas, coal mine methane, biogas (such as that derived from for example, the anaerobic digestion of biomass or alternatively from wastes, often also known as landfill gas), carbonaceous gas hydrates or clathrates, associated gas and refinery gas, is converted to syngas. Syngas is a mixture of hydrogen, carbon monoxide and carbon dioxide.

The syngas is then subjected to a bacterial fermentation to produce principally ethanol together with some minor by-products. Typical microorganisms utilised are acetogenic anaerobic bacteria especially such clostridium species as *Clostridium ljungdahlii, C. carboxydivorans, C. ragsdalei* and *C*. *autoethanogenum.*

The ethanol is then dehydrated, preferably catalytically, into ethylene.

The ethylene is then converted into an olefin derivative. Examples include solids such as polyethylene, liquids such as ethylbenzene or gases such as ethylene oxide. The olefin derivative may itselfbe further transformed for example in the case of ethylbenzene to styrene and then to polystyrene. Ethylene oxide can be transformed into ethylene glycol, ethanolamine, crown ethers and other materials which can again be subject to further transformation.

It has now surprisingly been found that by conducting the fermentation step in the same location as (i.e. within 5km of) the dehydration step and at least one conversion step unexpected benefits accrue especially in large scale plants. In particular in view of the differing energy, water and other chemical component requirements of each stage of the overall process it is possible to recycle by-product from one stage to another stage resulting in overall greater efficiency. By using acetogenic anaerobic bacteria fermenting syn-gas it is possible to convert relatively inexpensive raw materials into olefin derivatives on a large scale.

The process of the invention can however be practised where the fermentation step is not co-located with the dehydration and ethylene derivative process. In such instance it may be impractical for business reasons, such as lack of ethylene derivative market, to locate the dehydration unit and olefin conversion process at the same location as the supply and conversion of the carbonaceous feedstock to ethanol. In such instances product ethanol defined herein as the third product stream would be transported in bulk by road, rail or ship to a more suitable location for dehydration and ethylene conversion. Some stream integration benefits would be lost, however the advantages described hereinafter over other routes from carbonaceous feedstock to ethylene and ethylene derivatives would remain.

As an initial step syngas is prepared from a carbonaceous feedstock. Any suitable feedstock can be used. In the case of biomass feedstock being utilised several processes are known for converting such feeds into a methane containing biogas, one such well documented process is anaerobic digestion but others including pyrolysis could produce a gas suitable to be reformed to syngas. In addition biogas derived from waste disposal, known as landfill gas can be utilised by the process of the invention. In an embodiment of the invention biomass is converted into methane containing biogas by anaerobic digestion where the energy to maintain such a bacterial system at its optimal temperature usually above ambient conditions may come from heat evolved by exothermic steps elsewhere in the process as described hereinafter. The resulting biogas is then converted into syngas in known manner by reforming.. In general terms however biomass is not a preferred carbonaceous feedstock because it is not easily and cheaply available in large enough volumes for large-scale use. Additionally conversion of biomass to biogas can be inefficient in that few bacteria convert lignin into useful materials and there are thus large quantities of only partially digested wet feedstock to be disposed of.

In preferred embodiments the feedstock contains a substantial amount of methane.

The feedstock is fed to at least one reformer where it is converted to a mixture of hydrogen, carbon dioxide and carbon monoxide. A number of reforming processes are known. They include
1) steam methane reforming (SMR) in which the methane containing feedstock is reformed in an externally fired reformer in the presence of >2:1, preferably >2.5:1 molar steam : methane ratio.
2) autothermal reforming (ATR) in which the methane containing feedstock is reformed in the presence of steam and oxygen
3) partial oxidation (POX) in which the methane containing feedstock is reformed in the presence of oxygen and relatively low or zero concentrations of steam and
4) dry reforming in which the methane containing feedstock is reformed in an externally fired reformer in the presence of carbon dioxide and low or zero concentrations of feed steam.

A preferred reforming process is sulphur passivated reforming (SPARG) which is described in Hydrocarbon Processing, January 1986 p 71-74 and Oil and Gas Journal Mar 9, 1992, p 62-67. In a SPARG process sulphur is added to passivate the reforming catalyst. The sulphur reduces cokes formation, which can otherwise be a problem. It has been reported that the sulphur blocks large sites on the catalyst which are required to form coke but leaves the small sites open allowing reforming to continue.

The SPARG process is thought not to be widely used in the production of syngas. Reasons for this may include that most processes which use syngas require higher H2:CO ratios than are produced by SPARG reforming and because sulphur is generally a catalyst poison and thus needs to be removed from the syngas before it is used in many applications. Bacterial fermentations producing ethanol from syngas are generally tolerant of sulphur and so catalyst poisoning is not therefore a problem.

The different reforming processes give rise to a different mix of products with an SMR having an H₂: CO molar ratio in the range of about 3-5:1 compared with 2:1 for an ATR and 1.7 to 1.8:1 for a POX. An SMR typically produces syngas having a CO₂:CO molar ratio of 0.35:1 compared with 0.2:1 for an ATR and <0.1:1 for a POX. Dry gas reforming produces a feed gas with a reduced H₂:CO ratio and more effective conversion of CO₂ to CO than an SMR.

In some embodiments of the invention a plurality of reformers are used. In some embodiments of the invention the reformers are placed in series with the output from a first reactor optionally after treatment forming at least part of the input of a second reactor of the same or different type. An example of this is the so-called Lurgi Combined Reforming Process in which the methane containing feedstock, any recycled gases are fed to an SMR along with steam. The output from this reformer together, optionally, with unreacted feedstock and/or recycled gases are fed to an ATR. Our co-filed application ref 00205E describes the production of syngas in which initial feedstock optionally with recycle gases is first fed to an SMR, ATR or POX reformer. The output from that reformer optionally with unreacted feedstock and/or recycle gases is fed with steam to an ATR or POX reformer. By using a plurality of reactors in series the composition of the syngas can be different from that obtained from a single reactor and more suitable for further processing. Our co-filed application ref 00205E describes the production of syngas in which the feedstock and optionally recycle gas is feed to a plurality of reactors which may be the same or different which are at least partially arranged in parallel. In addition to allowing the product mix to be different from that obtained when a single reformer is used the arrangement produces redundancy. In the event that one reformer fails the redundancy allows syngas to continue to be produced. The culture used in the fermentation can thus be kept alive reducing or eliminating process plant down time.

The syngas exiting the reformers is cooled in one or more heat exchangers. The abstracted heat can be either transferred, in the form of steam, to other parts of the process or by direct exchange with streams from other parts of the overall process requiring to be heated by way of example the heat could be passed directly or by transfer of steam to the distillation reboilers to be described hereinafter. Our cofiled application reference 00202E describers such a process in more detail.

In a most preferred embodiment, the process of the present invention is operated at elevated pressure in both the reforming and fermentation steps. Preferably the pressure is in the range 2 to 20 barg for both steps. The pressure is preferably based on the optimum pressure for the fermentation step, and the reforming process operated at a suitably higher pressure to allow for inherent pressure loss between process steps as outlined previously, to provide the product stream at the required pressure for the fermentation step, and with minimal compression required for the recycle of the fourth gaseous product stream to the reforming process. One further advantage of SPARG technology, for example, is that it may be operated across a wide range of pressures dependent on the downstream processing required without significant variations in product distribution.

The fermentation process may use any suitable bacteria. The preferred fermentation process uses acetogenic anaerobic bacteria, especially a rod-shaped, gram positive, non-thermophilic anaerobe. Useful bacteria according to this invention include *Acetogenium kivui, Acetobacterium woodii, Acetoanaerobium noterae, Clostridium aceticum, Butyribacterium methylotrophicum, Clostridium acetobutylicum, Clostridium thermoaceticum, Eubacterium limosum, Peptostreptococcus productus, Clostridium ljungdahlii* and *Clostridium carboxydivorans.* In addition, other acetogenic anaerobic bacteria may be selected for use in the process of the invention. It will also be appreciated that a mixed culture of two or more bacteria may be used in the process of the present invention. Suitable microorganisms and/or growth conditions may include those disclosed in US 2007/0275447 which discloses a biologically pure culture of the microorganism *Clostridium carboxidivorans* having all of the identifying characteristics of ATCC no. BAA-624; and US2008/0057554 which discloses a biologically pure culture of the microorganism *Clostridium ragsdalei* having all of the identifying characteristics of ATCC No. BAA-622. A further particularly suitable bacteria is *Clostridium ljungdahlii,* especially suitable strains, or mixtures thereof, being *Clostridium ljungdahlii PETC, Clostridium ljungdahlii ERI2, Clostridium ljungdahlii C01, Clostridium ljungdahlii O-52. Clostridium ljungdahlii* and processes using such bacteria are described in DOE report "Bench-scale Demonstration of Biological Production of Ethanol from Coal Synthesis Gas", Topical Report 5, November 1995 (DOE Contract Number DE-AC22-92PC92118) and in patent applications WO 98/00558, WO 00/68407 and WO 02/08438. The fermentation process generally comprises contacting the product stream comprising CO, H₂ and CO₂ with the bacteria in the presence of a nutrient medium in a suitable reactor, for example a continuously stirred tank reactor (CSTR). It is clearly to be understood that the invention is not restricted to CSTRs and that other fermentation reactors can be used. Suitable temperatures and pressures are dependent on the bacteria and other process conditions used, but typical temperatures for the fermentation are between 25°C and 85°C, especially 35°C to 45°C and typical pressures are in the range atmospheric to 20 barg, preferably 2 to 17 barg. US 7 285 402 provides information about how to operate fermenters of use in the invention.

"Nutrient medium" is used generally to describe conventional bacterial growth media which contain vitamins and minerals sufficient to permit growth of a selected subject bacteria. Suitable nutrients are well-known, for example as described in US 7 285 402, WO 08/00558, US 5 807 722, US 5 593 886 and US 5 821 111.

The agitation rate may be selected depending on the reaction vessel and robustness of the bacteria. In particular, the reaction mixture is generally agitated at a suitable rate to ensure adequate gas dispersion and substantial avoidance of agglomeration of dispersed gas bubbles whilst minimising damage to the bacterium cells caused by any moving parts e.g. stirrer tips.

In practice this usually means that for a larger unit agitated with a stirrer a smaller RPM (revolutions per minute) is used than for a corresponding smaller unit (for a fixed RPM, the tip speed of a larger agitator is faster than that of a smaller agitator). Speeds of 20 to 1000 RPM are typical, with larger units operating at the lower rates.

The residence time may also be selected depending on the particulars of the reaction, and in order to obtain the desired conversion. The residence time is usually in the range 5 seconds to 20 minutes, and most typically in the range 10 seconds to 5 minutes.

Generally the fermentation step produces a gas phase product stream comprising CO, H₂ and CO₂ and a liquid phase comprising ethanol, water, bacteria, nutrients and by-products such as acetic acid and higher alcohols. Of these products the greatest mass is water. The liquid is typically filtered to retain cells and any other solids present within the fermenter and provides a filtered liquid phase for further processing. In addition, purging a portion of the fermenter broth containing bacterial cells in order to maintain a healthy cell culture is practiced. The filtered liquid is then treated to concentrate the ethanol. For example the liquid phase could be distilled to give a water/ethanol mix. Typically the recovered mixture comprises 40-95wt% ethanol. The bottoms comprising water, residual nutrients and other materials such as acetic acid are cooled. The cooled bottoms can be recycled entirely or partially back to the fermenter. The heat abstracted from the bottoms can be used elsewhere in the process. In some embodiments of the invention a plurality of distillation columns are used, preferred embodiments of this arrangement employ distillation columns that operate at different pressures. This means that the water/ethanol azeotrope boils at different temperatures in different columns and thus columns and operating pressures can be arranged such that the overheads from a first column act as a heat source for the reboiler of a second column. This multiple pairing of low pressure and high pressure columns allows ethanol separation at a very large scale and beyond that which may be practical to construct or transport as a single column. The rate of liquid feed to each column would be apportioned to achieve the desired optimum energy integration between the columns at their chosen operating pressures.

Components of the gas phase can be used in various ways. For example the gas phase can be recycled back to the reformer. Hydrogen could be separated from the mixture for example using a PSA. The separated hydrogen can be used either as a feedstock or as a fuel for example to heat a dehydrogenation process to be described hereinafter or to fire a reformer.

In general it will be necessary to purge a portion of the gas phase to prevent accumulation of inert gases such as nitrogen and argon. The purge may be before or preferably after separation of components from the gas phase. Conveniently the purged gases are passed to a burner and burnt. The heat can be used in other process steps.

The concentrated ethanol/water composition is then converted into ethylene in a dehydration process. Ethanol dehydration to ethylene is well known with references such as American Chemical Society Division of Fuel Chemistry 2007, 52, (2) p351-353 article describing historical context of use of fixed bed or fluid bed processes using alumina catalysts as well as exploration of alternative option of Fluid Catalytic Cracking with Y-zeolite catalysts either as sole feed or as a co-feed in an oil refinery context to generate ethylene. Morschbacker in Journal of Macromolecular science Part C: Polymer reviews 2009, 49, p79-84 also describes the manufacture of ethylene by the catalytic dehydration of ethanol derived from sugar cane ethanol and subsequent use for green polyethylene. A prominent comment by Morschbacker is that sugar derived ethanol suffers from price volatility and its conversion to ethylene is only economically viable at high oil prices. Thus a process which offers a price stable attractive route to ethylene would be desirable to the petrochemicals and olefins polymers industry. The process of the invention utilizing widely available gaseous carbonaceous feedstocks can provide such a route.

For example a part of the derived ethanol/water product could be further diluted with water typically to 50-90wt% ethanol. The water is preferably derived from other parts of the process for example as a co-product of the dehydration reaction. This feedstock is then vaporized and fed to a first dehydration reactor where the vaporized feedstock is passed over a catalyst such as γ-alumina to produce water and ethylene as primary products. In one embodiment, the additional water may be provided as steam and added after first vaporisation of the ethanol/water product. The conversion proceeds with good selectivity typically in excess of 85% to ethylene. In at least some embodiments further vaporised ethanol product optionally without added water is added to the materials exiting the first reactor and the resulting mixture passed to a second dehydration reactor where the vaporized feedstock is passed over a catalyst such as γ-alumina to produce water and ethylene as primary products. The conversion proceeds with good selectivity typically in excess of 85% to ethylene. In preferred embodiments of this variant the added ethanol is such that the ethanol/water ratio entering the second dehydration reactor is about the same as the ethanol/water ratio of the mixture entering the first dehydration reactor. In preferred embodiments the product leaving the first reactor plus additional feedstock is heated prior to entering the second dehydration reactor. In some embodiments the process is continued in like manner in a third and optionally further reactors. In preferred embodiments the ethylene product is purified and recovered by distillation at low temperature generating a product suitable for polymerization to polyethylene. Ethylene suitable for polyethylene manufacturing requires very high purity, typically 99.90 % with very low levels of any residual components such as hydrogen, methane, and ethane <2000ppm(vol) and total carbon oxides such as CO and CO₂ at approximately 1ppm(vol) level. In a most preferred embodiment recovered fractions of the reaction product stream which may comprise some ethylene arising from distillation separations, but may also comprise one or more of hydrogen, methane, ethane, propane ,propylene and various ethers may be utilized in a co-located plant arrangement as feed for the reformer or when not co-located for use as fuel gas. In a further most preferred embodiment a portion of the recovered water from the reactor product stream is recovered, optionally treated, and utilised in the fermentation process to produce ethanol when the overall process is practiced at the same location. In preferred embodiments of the invention dehydration is carried out a pressure in the range of 0.1 to 3MN/m² preferably in the range of in the range of 0.5 to 2MN/m².

In other embodiments of the invention ethanol is converted into ethylene and water by passing over zeolite catalysts in a fixed bed or most preferably a fluid bed reactor system.

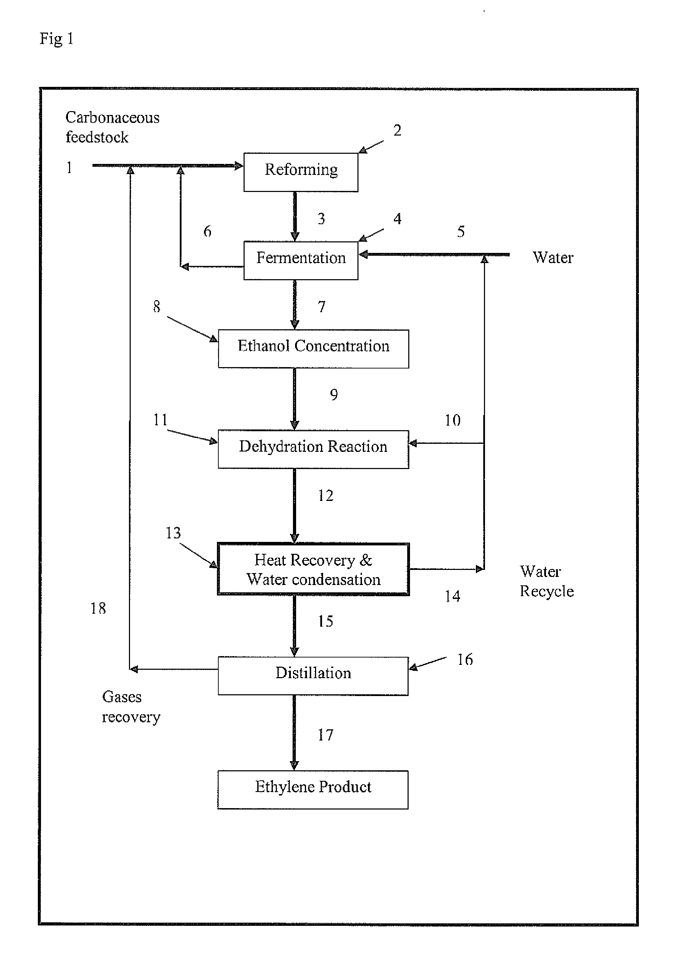

The process of the invention is illustrated in Fig 1. Carbonaceous feedstock is supplied from at least one source (not shown) via first conduit 1 to at least one reformer 2 as described herein. In the reformer or reformers 2 the carbonaceous feedstock is converted into a mixture comprising carbon dioxide, carbon monoxide and hydrogen. Other gases may be present also. The gases generally after cooling in a cooler (not shown) are then passed via second conduit 3 into one or more fermenters 4. Water is added via third conduit 5. Nutrients and other materials may be included in the water or feed or added in other ways. As explained herein the fermenter contains a bacterial culture which transforms at least a part of the gas entering the fermenter into ethanol.

Typically material exiting the fermenter comprises two streams; a gas stream and a liquid stream. The gas stream can comprise one or more of carbon monoxide, carbon dioxide, hydrogen and inert gases such as nitrogen. Other materials such as water and ethanol may be present typically in lesser amount. Gas exiting the fermenter is typically recycled back into the reformer by fourth conduit 6. While not illustrated in Fig 1 a small portion of the gas exiting the fermenter may be bled off to prevent build up of inerts. This material can conveniently be used as a fuel in one of the other steps such as firing an externally fired reformer, or for heating the concentration or dehydration stages or for powering the refrigeration stage to be described herein.

The liquid phase comprises a solution of ethanol in water along with other materials such as other metabolites such as alcohols and acetic acid, nutrients, cell debris. Solids such as cell debris can be removed by filters (not shown). The liquid is then passed by fifth conduit 7 to concentrator 8. Concentrator 8 may comprise one or more distillation columns. An ethanol enriched stream exits the concentrator via sixth conduit 9 and an ethanol depleted stream (not shown) is rejected. As explained herein the ethanol depleted stream may be recycled back to the fermenter.

The ethanol enriched stream optionally diluted with water supplied via seventh conduit 10 is then dehydrated in dehydrator 11 as described herein. Dehydration is generally conducted at elevated temperature and gives rises principally to water and ethylene together with smaller amounts of diethyl ether and other material. The dehydrator product passes via eighth conduit 12 to heat recovery and water condensation stage 13. As the product is cooled water condenses and is, for example run back to the fermentation stage by ninth conduit 14. Heat can be recovered and used elsewhere in the system.

Typically the product is cooled yet further until the ethylene condenses into liquid form. The condensed ethylene then passes via tenth conduit 15 to distillation stage 16. Purified ethylene exits the distillation stage 16 via eleventh conduit 17 for further use. The purified ethylene is typically very cold and may be used to cool the ethylene for distillation at an earlier stage. An impurities stream is rejected from the distillation column and may pass via twelfth conduit 18 to the carbonaceous feedstock. Alternatively or additionally the rejected impurities may be used a fuel in other stages. Typically the cool impurities stream passes through a heat exchanger (not shown) to cool ethylene for distillation.

In other embodiments of the invention the ethanol is converted into ethylene and water by passing over other catalysts such as tungstosilicic heteropolyacid catalysts in a fixed bed reactor system. An example of such a process is described in WO/2008138775A1.

The mixture exiting the dehydration step comprises water, ethylene and by-products such as diethyl ether as well as unreacted alcohol. The proportions of each component will depend on the type of catalyst being used and the exact reaction operating conditions of temperature and pressure. Ethylene has a very much lower boiling point than the water, diethyl ether or ethanol and is easily separated from them. These materials can be recirculated to the dehydration reactors. Alternatively or additionally the diethyl ether can be removed for use in applications or used as a fuel in other steps of this process.

The ethylene is then subjected to an olefin derivatisation step. The nature of this step depends on the required product.

In one embodiment of the invention the ethylene is converted into polyethylene. In this process the ethylene is purified for example by cryogenic distillation to give an ethylene stream and an impurities stream which may include materials such as hydrogen, carbon monoxide, carbon dioxide, alkanes and higher alkenes as well as some ethylene. The impurities stream can then be passed to the reforming stage where it can be used as a fuel or, more preferably, as a feedstock for conversion to syngas and thence to ethanol.

The ethylene stream is then polymerized to give polyethylene such as high density polyethylene (HDPE), low density polyethylene (LDPE) and linear low density polyethylene (LLDPE) for example using known methods such as the Innovene S and Innovene G processes licensed by INEOS Technologies. In addition to polyethylene the process produces heat which can be used in other parts of the process. The polyethylene produced is typically degassed to remove volatiles such as hydrogen and unreacted ethylene from the polymer. The ethylene can be separated and recycled into polymerisation step. At least some of the hydrogen and or carbon containing material obtained by degassing can be recycled to the reforming step as either or both of feedstock or fuel for an externally fired refonner.

The ethylene can be converted into other materials. For example it can be converted into ethylbenzene by acid catalysed reaction with benzene. The ethylbenzene so formed can be dehydrogenated to give styrene and hydrogen. Typically this is done by passing it admixed with 10 to 15 times its volume of steam over an iron oxide catalyst. The heat and steam for the process can be, at least in part, obtained from other parts of the process. The obtained hydrogen and by products such as toluene can be used as fuels or as feedstock for the reformer.

In other embodiments the ethylene is converted into ethylene oxide. For example, this can be done by passing the ethylene and oxygen over silver on alumina catalyst. The products include heat, carbon dioxide and steam together with ethylene oxide. The ethylene oxide can be lead away for further use for example producing ethylene glycol, ethanolamine and crown ethers. The remaining products can be re-utilised for example as feedstock for the reformer.

In further embodiments of the invention the ethylene is converted into ethylene dichloride by reaction with chlorine in the presence of iron (III) chloride. The ethylene chloride may be used as is or it may be converted into other materials such as vinyl chloride monomer (VCM) for further elaboration.

In still other embodiments the ethylene is converted into vinyl chloride monomer (VCM) by reaction with hydrogen chloride and a molecular oxygen containing gas over a copper chloride catalyst. This reaction is strongly exothermic and the heat produced can be used in other parts of the process for example the ethanol dehydration step.

The process of the invention provides a carbon efficient route to olefin derivatives in large volume. While in principle biomass provides a useful potential source of biogas for reforming in practice sufficiently large amounts of biogas derived from biomass are not readily or widely available to compete on similar scale terms with current petrochemical routes to olefin derivatives. By way of example typical new world-scale olefins units produce of the order of 1 million metric tones of ethylene per year or more. Alternative strategies especially those for converting a natural gas methane containing feedstock into ethylene include
i) the so-called MTO methanol to olefins process in which methane is first converted into methanol and thence into olefins
ii) conversion of methane via the Fischer-Tropsch process into FT naphtha. The FT naphtha is then cracked in a conventional olefins furnace to yield ethylene and other olefins. Oil and Gas European Magazine March 1st 2010 (pages 44-47) compares processes producing ethylene from coal, biomass and natural gas compared with conventional steam cracking of naphtha or light gases (ethane+ propane +butane) and steam cracking of FT derived Naphtha. It appears from this article that only about 35-40% of the product mixture obtained by either steam cracking of FT naphtha or via the MTO process is ethylene. It is instructive to compare the efficiency of the process of the invention in producing ethylene with that of a process for producing ethylene using MTO or of Fischer Tropsch liquids to olefins.

In MTO the first step is production of methanol from natural gas. Haldor Topsoe A/S estimate a carbon loop efficiency of up to 95% (see http://tinyurl.com/yabakxr). The obtained methanol is then converted by the MTO process to give a mixture of olefins including ethylene, propylene and butenes. C5 hydrocarbons and fuel gas are also produced. According to Barger et al Table 2 on page 112 of presentation entitled "Converting natural gas to ethylene and propylene using the UOP/Hydro MTO process" from Advanced catalytic science and Technology conference, Tokyo July 14-18, 2002 that ethylene is produced in equal mass as propylene at 882 metric te/d for a methanol feed of 5502 metric te/d giving 0.169 te ethylene yield per te methanol.

In the Fischer Tropsch process it is known that in a commercially worked process 330 x 10⁶ ft³d of lean methane rich gas produce 9 000 barrels/d of FT naphtha out of a total of 34 000 barrels of liquids. Since the density of ensuing FT naphtha is known to be about 700kgm⁻³ and the density of methane is known it is possible to deduce, knowing additionally that the upper yield of ethylene that can be expected from steam cracking of the naphtha is about 40%, that about 0.06 te ethylene are produced per te methanol.

### Example

Dry ethanol is firstly produced from a natural gas of composition as per Table 1 in co-pending application ref 00202E using a combined reforming process as described in that application. The results, as detailed in Column 2 of Table 2, of the aforementioned application states a figure of 0.922 te natural gas feedstock use per te of ethanol product.

This ethanol when passed into a dehydration process comprising 3 reactors, containing γ-alumina catalyst, in series each operating adiabatically at 2.0 MN/m³(gauge) with feed ethanol and water fed to 1^{st} reactor at 460°C with subsequent reheating of reaction product to 460°C to 2^{nd} reactor with final ethanol addition to 3^{rd} reactor at 440°C. Ethanol is apportioned as 30% by mass to 1^{st} reactor and 35% each to subsequent reactors and first reactor ethanol feed is mixed with recycle water at a water to ethanol ratio of 3.04 by mass. A portion of the product stream from the final reactor is partially cooled against recycle preheat to 1^{st} reactor and then ethanol feed vaporisation with a further portion being used to raise steam before recombining and being further cooled to a point below which water is condensed from the product stream. A portion, as required of this condensed water is recycled to the 1^{st} reactor vaporiser. Further cooling removes additional water from the gas stream, this condensed liquid fraction being subsequently stripped to remove residual hydrocarbons for use as fuel gas before its disposal. Gas conditioning for removal of undesirable oxygenates from the stream is incorporated before a final molecular sieve drying step on the non condensed fraction to remove any further residual water from the stream allows propylene refrigeration cooling to provide the reboiler duty for a low temperature fractionation tower which separates any C2 and C3 alkanes plus any C3 alkenes and heavier components as a base product stream with a liquid ethylene product taken from a side draw at top of the tower at -41C. Any light components present such as methane, hydrogen, carbon monoxide are removed from the tower in the condenser overheads stream. In this example, this light gas fraction has its heat recovered and is used as a component of the fuel gas for the furnace providing the energy to heat all the feeds for the rectors. The liquid ethylene from the fractionator is fed to a second stripping tower where purified ethylene suitable for polymerization is recovered from the base and a top fraction is recovered as vapour at -30 C and returned to the fractionator. The ethylene fraction recovered from the process as described above is 57.9% by mass of the ethanol fed to the reactors.

Thus overall, it is derived in this non integrated example that approximately 1.6 te natural gas /te ethylene is required by the process of the invention.

Comparison of different commercial methods for producing ethylene from natural gas is summarized in the attached Table.

| | Invention | MTO | Fischer Tropsch & Steam Cracking |
|---|---|---|---|
| Natural gas te/ Ethylene te | 1.6 | 3.12 | 16.8 |

It will therefore be apparent that the invention produces ethylene and ethylene derivatives far more efficiently in terms of mass of product per unit mass of methane containing feedstock than other processes.

Furthermore by integrating the process ie by conducting the reforming process, the fermentation process, the dehydration process and optionally at least one olefin derivatisation step in the same location (ie within 5 km preferably within 4 km of each other more preferably within 3km of each other) it is possible to utilise efficiently materials such as water and energy and by-products since waste from one step can be used in a further step. Such integration will only serve to enhance the presented result in above table by driving down the natural gas feedstock use further as recovered hydrocarbon and carbon monoxide gases are recycled back to the reformer, thus reducing the requirement for fresh natural gas.

## Claims

1. A process for producing ethylene from a carbonaceous containing feedstock the process comprising the steps of
a) reforming the carbonaceous containing feedstock into a first product stream comprising carbon dioxide, carbon monoxide and hydrogen;
b) fermenting the first product stream with an acetogenic anaerobic bacteria to produce a second product stream comprising ethanol;
c) concentrating ethanol from the second product stream into a third product stream; and optionally
d) dehydrating ethanol in the third product stream to produce ethylene.

2. A process for producing an olefin derivative the process comprising producing ethylene by a process as claimed in claim 1 and
e) converting the ethylene into an olefin derivative.

3. A process as claimed in claim 1 wherein the steps a) to c) are performed within 5 km of each other.

4. A process as claimed in claim 3 wherein steps a) to d) are performed within 5km of each other.

5. A process as claimed in any one of the preceding claims wherein the process produces at least 200 te/d ethylene.

6. A process as claimed in any one of the preceding claims wherein less than 2.5 te, preferably less than 2 te methane equivalent feed stock is used per te of ethylene produced.

7. A process as claimed in claim 2 wherein step e) comprises polymerizing ethylene to form polyethylene.

8. A process as claimed in claim 2 wherein step e) comprises the step of converting ethylene to ethylene oxide.

9. A process as claimed in claim 8 wherein the ethylene oxide is converted into ethylene glycol.

10. A process as claim in claim 2 wherein step e) comprises the step of converting ethylene to ethylene dichloride.

11. A process as claimed in claim 2 wherein step e) comprises the step of converting ethylene to ethylbenzene.

12. A process as claimed in any one of the preceding claims wherein step d) is performed at a pressure in the range of 0.1 to 3MN/m².

13. A process as claimed in any one of the preceding claims wherein the dehydration to ethylene step d) is performed in a plurality of reactors.

14. A process as claimed in any one of the preceding claims wherein water produced in step d) is used, optionally after treatment, in step b).

15. A process as claimed in any one of the preceding claims wherein product exiting step d) comprises a first, ethylene enriched, fraction and a second, ethylene depleted, fraction.

16. A process as claimed in claim 15 wherein at least a part of the second, ethylene depleted, fraction is used as a carbonaceous feed stock.

17. A process as claimed in any one of the preceding claims wherein the carbonaceous feedstock is at least partially obtained by anaerobic digestion of biomass.

18. A process as claimed in any one of the preceding claims wherein step c) comprises the step of distillation in a plurality of distillation stages arranged in parallel operating at different pressures such that distillate passing from a first distillation stage heats bottoms of a second distillation stage.

19. Apparatus for producing ethylene comprising
i) a reformer for reforming a carbonaceous feedstock into a first product stream comprising carbon dioxide, carbon monoxide and hydrogen;
ii) a fermenter for fermenting the first product stream into a second product stream comprising ethanol;
iii) means for concentrating ethanol from the second product stream; and optionally
iv) a dehydrator for dehydrating the ethanol to ethylene.

20. The use of at least one pair of parallel distillation stages in recovering ethanol from a distillation feed mixture comprising ethanol at less than 10% by weight, acetic acid, and fermentation nutrient residues generated by a non-yeast bacterial fermentation such as a syngas fermentation, at least one pair of distillation stages comprising a first distillation stage operating at a first temperature and a first pressure and a second distillation stage operating at a operating at a second temperature and a second pressure such that distillate passing from the first distillation stage provides the heat for the bottoms reboiler of the second distillation stage.
